# EUROPEAN PATENT APPLICATION

(11) **EP 4 736 808 A1**
(43) Date of publication of application: **06.05.2026**
(21) Application number: 25212266.8
(22) Date of filing: 30.10.2025
(51) Int. Cl.: A61C 13/00, A61C 13/083, C04B 35/00

(54) **ZIRCONIA PORCELAIN BLOCK, PREPARATION METHOD AND APPLICATION THEREOF**

(30) Priority: 01.11.2024 CN 202411545893
(71) Applicant: Shenzen Yurucheng Dental Materials Co., Ltd., Shenzhen, Pingshan 518000 (CN)
(72) Inventor: LI, Zongyu, Shenzhen, 518000 (CN); LIU, Wei, Shenzhen, 518000 (CN); ZHANG, Yu, Shenzhen, 518000 (CN); YANG, Qiangwen, Shenzhen, 518000 (CN); ZHANG, Xu, Shenzhen, 518000 (CN); LIU, Jianjun, Shenzhen, 518000 (CN)
(74) Representative: Monteiro Alves, Inês

(57) **Abstract**

A zirconia porcelain block, a preparation method and an application thereof are provided. The zirconia porcelain block includes an inner zirconia block and an outer zirconia block wrapping the inner zirconia block. The outer zirconia block includes a plurality of zirconia layers provided vertically in order of transparency, and the thickness of the zirconia layer with the maximum transparency and the thickness of the zirconia layer with the minimum transparency are both greater than the thickness of the zirconia layers between the maximum transparency and minimum transparency. The transparency of the inner zirconia block is the same as the maximum transparency of the plurality of zirconia layers, and the bottom end of the inner zirconia block is located in the zirconia layer with the minimum transparency. The height of the inner zirconia block is not less than two-thirds of the height of the outer zirconia block.

## Description

### TECHNICAL FIELD

The present application relates to the technical field of denture materials, and in particular to a zirconia porcelain block, a preparation method and an application thereof.

### BACKGROUND

Human teeth consist of a plurality of layers of structures from the inside out, including pulp, dentin and enamel. The strength of dentin is lower than that of enamel. Therefore, during normal chewing and abrasion, the dentin can buffer the wear of enamel, thus preventing teeth from being broken by sudden huge force. The material currently used for mid-end and low-end dental zirconia all-porcelain restorations is a single homogeneous material, which has the same strength from the inside to the outside. Without the buffering dentin, the service life of the denture is reduced. In appearance, it also shows certain color and transparency, which cannot well simulate the aesthetics of the gradient of teeth and the mechanical effects of different hardness inside and outside the teeth.

### SUMMARY

In order to solve existing shortcomings, the present application provides a zirconia porcelain block, a preparation method and an application thereof.

The technical solution adopted by the present application to solve the technical problems is as follows. The zirconia porcelain block includes an inner zirconia block made of a micron-sized zirconia mixture and an outer zirconia block made of a nanozirconia mixture wrapping the inner zirconia block. The outer zirconia block includes a plurality of zirconia layers provided sequentially in a vertical direction in order of transparency, a thickness of the zirconia layer with maximum transparency and a thickness of the zirconia layer with minimum transparency both are greater than a thickness of the zirconia layer between the maximum transparency and the minimum transparency; and a transparency of the inner zirconia block is the same as maximum transparency of the plurality of zirconia layers, a bottom end of the inner zirconia block is located in the zirconia layer with the minimum transparency, and a height of the inner zirconia block is no less than two-thirds of a height of the outer zirconia block.

In an embodiment, the inner zirconia block is a zirconia block with an arched cross section.

In an embodiment, the inner zirconia block is made of a zirconia mixture with a particle size between 0.2 µm to 0.5 µm, and the outer zirconia block is made of a zirconia mixture with a particle size between 10 nm to 80 nm.

In an embodiment, the zirconia mixture of the outer zirconia block includes high light-transmitting powder with transparency of 57% and low light-transmitting powder with transparency of 43%, and each of the zirconia layers is made by mixing the high light-transmitting powder and the low light-transmitting powder in a certain proportion.

In an embodiment, the zirconia mixture includes the following components in parts by weight: 90 to 95 parts of ZrO₂, 5 to 8 parts of Y₂O₃, 0.01 to 0.3 parts of Fe₂O₃, 0.01 to 0.3 parts of Er₂O₃, 0 to 0.2 parts of Mn₂O₃ and 0 to 0.5 parts of Al₂O₃.

In an embodiment, the thickness of the zirconia layer with the maximum transparency and the thickness of the zirconia layer with the minimum transparency among the plurality of zirconia layers are the same.

The present application further provides a method for preparing a zirconia porcelain block, including:

obtaining inner layer zirconia powder of an inner zirconia block having same maximum transparency as outer layer zirconia powder, and pre-pressing the inner layer zirconia powder to form an inner zirconia green body;

obtaining outer layer zirconia powder with different transparencies for preparing a zirconia layer, providing sequentially a plurality of zirconia layers wrapping the inner zirconia green body from bottom to top in an axial direction of the inner zirconia green body in order of transparency of the outer zirconia powder from small to large, and dry-pressing the inner zirconia green body and the plurality of zirconia layers as a whole to form a zirconia porcelain block green body;
cold isostatic pressing the zirconia porcelain block green body; and
sintering the zirconia porcelain block green body after cold isostatic pressing to obtain the zirconia porcelain block.

In an embodiment, a pre-pressing pressure of the inner zirconia green body is no greater than 5 MPa, an overall dry- pressing pressure is 60 MPa to 70 MPa, and a pressure maintaining time is 10 seconds to 40 seconds; and
a cold isostatic pressing pressure is 100 MPa to 300 MPa, a pressure maintaining time is 10 minutes to 15 minutes, and a sintering temperature is 1000°C to 1100°C.

In an embodiment, a thickness of the zirconia layer with maximum transparency and a thickness of the zirconia layer with minimum transparency among the plurality of zirconia layers are both greater than a thickness of the zirconia layers between the maximum transparency and the minimum transparency, and a height of the inner zirconia block is no less than two-thirds of a sum of heights of the plurality of zirconia layers.

The present application further provides a denture made of the zirconia porcelain block as described above.

The beneficial effects of the present application are as follows. The outer zirconia block and the inner zirconia of the present application adopt a mixture of zirconia of different particle sizes to form two structures with different hardnesses, thereby improving the service life and comfort of the denture, and better simulating the structural characteristics of real teeth. The multi-layer structure of the outer zirconia block can better realize the changes in the color and transparency of real teeth, thereby improving the aesthetics of the denture.

### BRIEF DESCRIPTION OF THE DRAWINGS

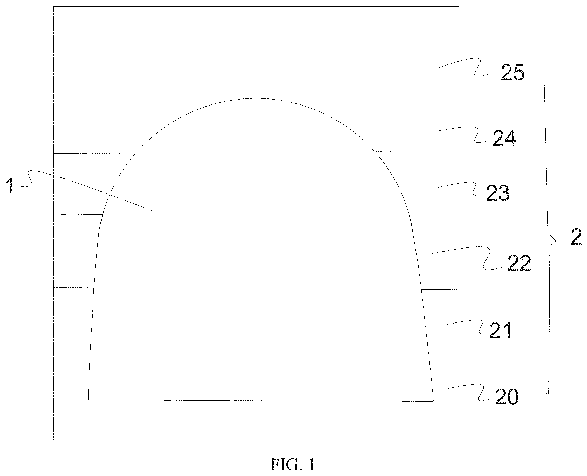

FIG. 1 is a schematic view of the sectional structure of a zirconia porcelain block according to an embodiment of the present application.

Description of the reference signs:
1. inner zirconia block; 2. outer zirconia block; 20. first zirconia layer; 21. second zirconia layer; 22. third zirconia layer; 23. fourth zirconia layer; 24. fifth zirconia layer; 25. sixth zirconia layer.

### DETAILED DESCRIPTION OF THE EMBODIMENTS

In order to more clearly illustrate the purpose, technical solutions and advantages of the embodiments of the present application, the present application will be further described below in conjunction with the embodiments for a clear and complete description. Obviously, the embodiments described are only some embodiments of the present application, not all embodiments. Based on the embodiments of the present application, all other embodiments obtained by ordinary technicians in this field without creative work are within the scope of protection of the present application.

As shown in FIG. 1, a zirconia porcelain block includes an inner zirconia block 1 made of a micron-sized zirconia mixture and an outer zirconia block 2 made of a nanozirconia mixture wrapping the inner zirconia block 1. That is, the inner zirconia block 1 is prepared using a micron-sized zirconia mixture, and a zirconia mixture with a particle size between 0.2 to 0.5 µm is configured to prepare the micron-sized zirconia mixture. The outer zirconia block 2 is prepared using a nanozirconia mixture, and the hardness of the outer zirconia block 2 prepared in this way is greater than the hardness of the inner zirconia block 1. The zirconia mixture with a particle size between 10 nm to 80 nm is configured to prepare the outer zirconia block 2. At this time, the zirconia mixture includes the following components in parts by weight: 90 to 95 parts of ZrO₂, 5 to 8 parts of Y₂O₃, 0.01 to 0.3 parts of Fe₂O₃, 0.01 to 0.3 parts of Er₂O₃, 0 to 0.2 parts of Mn₂O₃ and 0 to 0.5 parts of Al₂O₃. By adjusting the content of each component, a zirconia mixture can be formed to prepare ceramic blocks with different transparencies. Compared with the high translucency zirconia mixture, the low translucency zirconia mixture has a higher content of ZrO₂ and a lower content of Y₂O₃, and the amount of Fe₂O₃, Er₂O₃, Al₂O₃ and Mn₂O₃ in the zirconia mixtures of different translucency is basically the same. At this time, firstly, mixing high translucency powder with transparency of 57% and low translucency powder with transparency of 43% to prepare high translucency powder with transparency of 57% and low translucency powder with transparency of 43%. Then, mixing the high translucency powder and the low translucency powder in a certain proportion to prepare zirconia layers of different translucency. For example, when four zirconium oxide layers are provided, the mass percentages of high-transmitting powder and low-transmitting powder in each zirconium layer are 20% and 80%, 40% and 60%, 60% and 40%, and 80% and 20% in order of transparency from small to large. When the zirconium oxide layer is set to five, six, seven, and eight layers, the same principle can be used to mix and prepare. The outer zirconia block 2 includes a plurality of zirconia layers provided sequentially in the vertical direction in order of transparency, and the thickness of the zirconia layer with maximum transparency and the thickness of the zirconia layer with minimum transparency are both greater than the thickness of the zirconia layer between the maximum transparency and the minimum transparency. That is, the outer zirconia block 2 includes a plurality of zirconia layers, the transparency of each zirconia layer is different, such as providing the bottommost zirconia layer as the zirconia layer with minimum transparency, and providing the topmost zirconia layer as the zirconia layer with the maximum transparency, and the thickness of the topmost zirconia layer and topmost zirconia layer are both greater than the thickness of the middle zirconia layer. In an embodiment, the thickness of the zirconia layer with the maximum transparency among the plurality of zirconia layers is the same as the thickness of the zirconia layer with the minimum transparency, that is, the thickness of the topmost zirconia layer and the thickness of the bottommost zirconia layer are the same. The transparency of the inner zirconia block 1 is the same as the maximum transparency among the plurality of zirconia layers, the bottom end of the inner zirconia block 1 is located in the zirconia layer with the minimum transparency, and the height of the inner zirconia block 1 is not less than two-thirds of the height of the outer zirconia block 2, which more realistically simulates the structure of real teeth.

In an embodiment, as shown in FIG. 1, the inner zirconia block 1 is a zirconia block with an arched cross-section. That is, the inner zirconia block 1 is a spherical-shaped structure, the bottom surface of the inner zirconia block 1 corresponds to the zirconia layer with the minimum transparency, and the dome of the inner zirconia block 1 corresponds to any other zirconia layer according to the situation. In this way, there will be no protruding edges and sharp corners that generate stress, and the force on the outer zirconia block can be better buffered.

A method for preparing a zirconia porcelain block includes following steps:

Obtaining inner zirconia powder of an inner zirconia block 1 having same maximum transparency as outer zirconia powder, and pre-pressing the inner zirconia powder to form an inner zirconia green body. The raw materials used include the following components in parts by weight: 90 to 95 parts of ZrO₂, 5 to 8 parts of Y₂O₃, 0.01 to 0.3 parts of Fe₂O₃, 0.01 to 0.3 parts of Er₂O₃, 0 to 0.2 parts of Mn₂O₃ and 0 to 0.5 parts of Al₂O₃. The particle sizes of all components are between 0.2-0.5 µm. The pre-pressing pressure is no greater than 5 MPa. Since the particle sizes of all components are larger than the particle sizes of the outer zirconia powder, the transparency is required to be the same as the maximum transparency of the outer zirconia powder, and the amount of ZrO₂ is reduced while the amount of Y₂O₃ is increased.

Obtaining outer layer zirconia powder with different transparencies for preparing a zirconia layer, and providing sequentially a plurality of zirconia layers wrapping the inner zirconia green body from bottom to top in an axial direction of the inner zirconia green body in order of transparency of the outer zirconia powder from small to large, and dry-pressing the inner zirconia green body and the plurality of zirconia layers as a whole to form a zirconia porcelain block green body. The transparency of the outer zirconia powder is the same as the transparency of the zirconia layers formed thereby. At this time, the plurality of zirconia layers are stacked in the vertical direction, and the transparency of each zirconia layer is different, while the inner zirconia green body is wrapped by the plurality of zirconia layers. When the inner zirconia block 1 is a zirconia block with an arched cross section, the bottom surface of the inner zirconia block 1 corresponds to the zirconia layer with the minimum transparency. Firstly, weighing the amount of each component in the raw material in proportion, then mixing the raw materials to produce high light-transmitting powder with transparency of 57% and low light-transmitting powder with transparency of 43% for preparing a zirconia layer, and then mixing the high light-transmitting powder and the low light-transmitting powder in a certain proportion to prepare outer layer of zirconia powder for preparing zirconia layers with different transparencies. The raw materials used include the following components in parts by weight: 90 to 95 parts of ZrO₂, 5 to 8 parts of Y₂O₃, 0.01 to 0.3 parts of Fe₂O₃, 0.01 to 0.3 parts of Er₂O₃, 0 to 0.2 parts of Mn₂O₃ and 0 to 0.5 parts of Al₂O₃. The principle of adjustment is that the low transparency zirconia mixture has a higher content of ZrO₂ and a lower content of Y₂O₃ than the high transparency zirconia mixture, while the amount of Fe₂O₃, Er₂O₃, Al₂O₃ and Mn₂O₃ in the zirconia mixtures with different transparencies is basically the same. The particle size of all components in the raw material is between 10 nm to 80 nm. The overall dry-pressing pressure is 60 Mpa to 70 Mpa, and the pressure maintaining time is 10 S to 40 S. In an embodiment, the thickness of the zirconia layer with the maximum transparency and the thickness of the zirconia layer with the minimum transparency in the plurality of zirconia layers are both provided to be greater than the thickness of the zirconia layer between the maximum transparency and the minimum transparency, so that the structural strength of the denture can be ensured when the porcelain block is processed into a denture. The height of the inner zirconia block 1 is not less than two-thirds of the sum of the heights of the plurality of zirconia layers, and the inner zirconia block 1 can better play a buffering role.

Cold isostatic pressing the zirconia porcelain block green body, the pressure of the cold isostatic pressing is 100 MP to 300 MPa, and the pressure maintaining time is 10 minutes to 15 minutes.

Sintering the zirconia porcelain block green body after cold isostatic pressing to obtain the zirconia porcelain block, the sintering temperature is 1000 °C to 1100°C, and the heating rate of is no more than 2°C/min.

A denture is made of the zirconia porcelain block described above.

### Example 1

Weighing and mixing 90 parts of ZrO₂, 8 parts of Y₂O₃, 0.15 parts of Fe₂O₃, 0.3 parts of Er₂O₃, 0.015 parts of Mn₂O₃, and 0.1 parts of Al₂O₃ with particle size of 0.3 µm to produce inner layer zirconia powder with transparency of 46% for preparing the zirconia layer.

Weighing and mixing 94 parts of ZrO₂, 5 parts of Y₂O₃, 0.09 parts of Fe₂O₃, 0.3 parts of Er₂O₃, 0.02 parts of Mn₂O₃, and 0.05 parts of Al₂O₃ with particle size of 50 nm to produce low light-transmitting powder with transparency of 43% for preparing the zirconia layer.

Weighing and mixing 91 parts of ZrO₂, 7 parts of Y₂O₃, 0.1 parts of Fe₂O₃, 0.4 parts of Er₂O₃, and 0.1 parts of Al₂O₃ with particle size of 50 nm to produce high light-transmitting powder with transparency of 57% for preparing the zirconia layer.

Preparing the outer layer zirconia powder for preparing each zirconia layer by mixing high light-transmitting powder with transparency of 57% and low light-transmitting powder with transparency of 43% in each zirconia layer in the mass percentages of 20% and 80%, 30% and 70%, 40% and 60%, 60% and 40%, 70% and 30%, and 80% and 20%, respectively. As shown in FIG. 1, a first zirconia layer 20, a second zirconia layer 21, a third zirconia layer 22, a fourth zirconia layer 23, a fifth zirconia layer 24 and a sixth zirconia layer 25 are formed accordingly. At this time, in the total weight of the plurality of zirconia layers, the first zirconia layer 20 and the sixth zirconia layer 25 each account for 20% of the total weight, and the other zirconia layers each account for 15% of the total weight.

Preparing the zirconia porcelain block body using a dry-pressing mold. Firstly, pre-pressing the inner layer of zirconia powder at pressure of 5 MPa by using a mold to form an inner zirconia green body. Then, providing a portion of the outer layer of zirconia powder of the first zirconia layer 20 in another mold. Then, placing the inner zirconia body in the middle of the first zirconia layer 20. Then, providing the outer layer of zirconia powder of the remaining first zirconia layer 20 around the circumference direction of the inner zirconia body. Finally, providing sequentially the zirconia layer 21, the third zirconia layer 22, the fourth zirconia layer 23, the fifth zirconia layer 24 and the sixth zirconia layer 25 on the first zirconia layer 20 to wrap the inner zirconia green body. After each outer layer of zirconia powder is provided, it is pre-pressed, and the pre-pressing pressure is not greater than 5 MPa. If the pre-pressing pressure of 5 MPa is selected, each outer layer of zirconia powder forms a zirconia layer. After providing, dry-pressing the inner zirconia green body and each zirconia layer as a whole to form a zirconia porcelain block green body. The pressure is 70 Mpa and the pressure maintaining time is 30 S.

Cold isostatic pressing the zirconia porcelain block green body, the pressure of the cold isostatic pressing is 270 MPa, and the pressure maintaining time of 15 minutes.

Sintering the zirconia porcelain block green body after cold isostatic pressing to obtain the zirconia porcelain block, the sintering temperature is 1050°C, and the heating rate is no more than 2°C/min.

Carving the zirconia porcelain blocks to form a denture. The denture has an appropriate cutting end size, natural color transition, uniform light transparency and strength. The flexural strength of the first zirconia layer 20, the second zirconia layer 21, the third zirconia layer 22, the fourth zirconia layer 23, the fifth zirconia layer 24 and the sixth zirconia layer 25 is 960 MPa, 950 MPa, 945 MPa, 940 MPa, 930 MPa and 920 MPa, respectively, and the corresponding transparency is 46%, 48%, 50%, 53%, 54% and 56%.

It should be understood that those skilled in the art may make improvements or changes based on the above description, and all such improvements and changes should fall within the scope of protection of the present application.

## Claims

1. A zirconia porcelain block, **characterized by** comprising:
an inner zirconia block made of a micron-sized zirconia mixture and an outer zirconia block made of a nanozirconia mixture wrapping the inner zirconia block;
wherein the outer zirconia block comprises a plurality of zirconia layers provided sequentially in a vertical direction in order of transparency, a thickness of the zirconia layer with maximum transparency and a thickness of the zirconia layer with minimum transparency both are greater than a thickness of the zirconia layer between the maximum transparency and the minimum transparency; and
a transparency of the inner zirconia block is the same as maximum transparency of the plurality of zirconia layers, a bottom end of the inner zirconia block is located in the zirconia layer with the minimum transparency, and a height of the inner zirconia block is no less than two-thirds of a height of the outer zirconia block.

2. The zirconia porcelain block according to claim 1, wherein the inner zirconia block is a zirconia block with an arched cross section.

3. The zirconia porcelain block according to claim 1, wherein the inner zirconia block is made of a zirconia mixture with a particle size between 0.2 µm to 0.5 µm, and the outer zirconia block is made of a zirconia mixture with a particle size between 10 nm to 80 nm.

4. The zirconia porcelain block according to claim 3, wherein the zirconia mixture of the outer zirconia block comprises high light-transmitting powder with transparency of 57% and low light-transmitting powder with transparency of 43%, and each of the zirconia layers is made by mixing the high light-transmitting powder and the low light-transmitting powder in a certain proportion.

5. The zirconia porcelain block according to claim 4, wherein the zirconia mixture comprises following components in parts by weight: 90 to 95 parts of ZrO₂, 5 to 8 parts of Y₂O₃, 0.01 to 0.3 parts of Fe₂O₃, 0.01 to 0.3 parts of Er₂O₃, 0 to 0.2 parts of Mn₂O₃ and 0 to 0.5 parts of Al₂O₃.

6. The zirconia porcelain block according to claim 1, wherein the thickness of the zirconia layer with the maximum transparency and the thickness of the zirconia layer with the minimum transparency among the plurality of zirconia layers are the same.

7. A method for preparing a zirconia porcelain block, **characterized by** comprising:
obtaining inner layer zirconia powder of an inner zirconia block having same maximum transparency as outer layer zirconia powder, and pre-pressing the inner layer zirconia powder to form an inner zirconia green body;
obtaining outer layer zirconia powder with different transparencies for preparing a zirconia layer, providing sequentially a plurality of zirconia layers wrapping the inner zirconia green body from bottom to top in an axial direction of the inner zirconia green body in order of transparency of the outer zirconia powder from small to large, and dry-pressing the inner zirconia green body and the plurality of zirconia layers as a whole to form a zirconia porcelain block green body;
cold isostatic pressing the zirconia porcelain block green body; and
sintering the zirconia porcelain block green body after cold isostatic pressing to obtain the zirconia porcelain block.

8. The method for preparing the zirconia porcelain block according to claim 7, wherein a pre-pressing pressure of the inner zirconia green body is no greater than 5 MPa, an overall dry-pressing pressure is 60 MPa to 70 MPa, and a pressure maintaining time is 10 seconds to 40 seconds; and
a cold isostatic pressing pressure is 100 MPa to 300 MPa, a pressure maintaining time is 10 minutes to 15 minutes, and a sintering temperature is 1000°C to 1100°C.

9. The method for preparing the zirconia porcelain block according to claim 7, wherein a thickness of the zirconia layer with maximum transparency and a thickness of the zirconia layer with minimum transparency among the plurality of zirconia layers are both greater than a thickness of the zirconia layers between the maximum transparency and the minimum transparency, and a height of the inner zirconia block is no less than two-thirds of a sum of heights of the plurality of zirconia layers.

10. A denture made of the zirconia porcelain block according to any one of claims 1-6.
